# EUROPEAN PATENT APPLICATION

(11) **EP 3 656 874 A1**
(43) Date of publication of application: **27.05.2020**
(21) Application number: 18461629.0
(22) Date of filing: 26.11.2018
(51) Int. Cl.: C12Q 1/6848, C12Q 1/689, G16B 25/20

(54) **A METHOD OF SELECTING PCR PRIMERS, PREFERABLY FOR DETECTION OF ANTIBIOTIC RESISTANCE GENES**

(71) Applicant: Uniwersytet Warszawski, 00-927 Warszawa (PL)
(72) Inventor: Gorecki, Adrian, 04-036 Warszawa (PL); Dziurzynski, Mikolaj, 01-142 Warszawa (PL); Decewicz, Przemyslaw, 07-221 Branszczyk (PL); Dziewit, Lukasz, 02-389 Warszawa (PL)
(74) Representative: AOMB Polska Sp. z.o.o.

(57) **Abstract**

The invention is related to a method of selecting PCR primers, preferably for detection of antibiotic resistance genes, comprising the following computer-implemented steps:
a) providing a first reference gene sequence database comprising first gene sequences;
b) providing a second reference gene sequence database comprising second gene sequences;
c) searching said first gene sequences in said second reference gene sequence database;
d) creating a third redundant gene sequence database comprising the gene sequences found as the result of step c);
e) creating a third non-redundant gene sequence database by selecting all and only unique gene sequences from said third redundant gene sequence database;
f) providing a test primer database, comprising primer pairs for amplifying gene sequences;
g) performing *in silico* PCR using as a matrix said second reference gene sequence database and said test primer database;
h) counting the number P of products obtained in the step g) for each primer pair from said test primer database;
i) for each of the P products, searching for it in said third redundant gene sequence database and counting the number of products found therein and correct products CP;
j) performing *in silico* PCR using as a matrix said third non-redundant gene sequence database and said test primer database;
k) counting the number RP of reference products obtained in the step j) for each primer pair from said test primer database;
l) for each primer pair from said test primer database: calculating the taxonomic diversity of products as the number PT of taxons from which the products of step g) originated and the reference taxonomic diversity as the number RT of taxons corresponding to all variants of a particular gene sequence from said first reference gene sequence database found in said third redundant gene sequence database;
m) for each primer pair from said test primer database:
calculating the primers specificity parameter S as the ratio of correct products CP to the number P of products obtained in the step g); S=CP/P;
calculating the primers efficacy parameter E as the ratio of reference products RP to the number NRER of the non-redundant variants of a particular gene in said third non-redundant gene sequence database; E=RP/NRER;
calculating the primers taxonomic efficacy parameter TE as the ratio of the taxonomic diversity of products PT to the reference taxonomic diversity RT; TE=PT/RT;
n) for each primer pair from said test primer database: assigning parameters of the primers specificity parameter S, the primers efficacy parameter E and the primers taxonomic efficacy parameter TE to said primer pair;
o) selecting primer pairs according to their values of the primers specificity parameter S and/or the primers efficacy parameter E and/or the primers taxonomic efficacy parameter TE.

## Description

### Technical Field

The invention is related to a method of selecting PCR primers, preferably for detection of antibiotic resistance genes. The method is performed in computer implemented steps (*in silico*) and comprises operation on gene sequence databases and PCR primers database. However, the gene sequence databases and the primer database are populated with data taken from biological samples. Likewise, the PCR primers selected according to this method, can be applied in a laboratory environment to amplify target gene sequences.

### Background

Antibiotics are considered the most effective pharmaceuticals used in treatment of bacterial infectious diseases. Since 1930's, antibiotics have saved millions of lives, and the positive influence of these pharmaceuticals on overall public health cannot be overrated (1). The improvement of modern technologies and laboratory techniques led to discovery and production of many synthetically modified derivatives of already known antibiotics and resulted in a development of many fully synthetic, broad-spectrum antibiotics that have no equivalents in natural environments (e.g. sulfonamides and fluoroquinolones) (2). However, in the last 30 years, only one really novel antibiotic (i.e. teixobactin) was discovered (3).

The great success of antibiotics in treating infectious diseases in medicine prompted their wide use also in several other fields. This extensive usage has already resulted in millions of metric tons of antibiotics used throughout 60 years after their first introduction (4). Various classes of antibiotics, either as therapeutic or prophylactic agents, are widely used in agricultural practice or as growth promoters in animals farming (5-6). Moreover, there are also many evidences for antibiotics overuse and misuse in medicine and veterinary practice (7-8). Unfortunately, high market demands coupled with a not strictly controlled usage of these pharmaceuticals led to accelerated development and spread of antibiotic resistance genes (ARGs) in bacteria. Antibiotic resistant bacteria, antibiotic resistance genes and antibiotics, are now considered a novel class of emerging pollutants that pose a serious risk for both animal and public health (9-10).

Bacterial resistance to antibiotics could be "intrinsic" and "non-specific", which is mainly a consequence of the cell-wall structure (11), type of cellular respiration (12) or production of bacterial capsules (13). On the other hand, the "specific" resistance is linked with a presence of ARGs, that become more and more widespread. What makes antibiotic resistance genes so abundant, is their high mobility within bacterial microbiocenoses, as ARGs easily disseminate by hitchhiking mobile genetic elements (MGEs) such as plasmids, integrons and transposable elements (14). MGEs carrying antibiotic resistance genes can translocate between various, even taxonomically remote strains of bacteria, and therefore this type of horizontal gene transfer is considered to be the most important factor, responsible for the spread of ARGs in the environment (15). Moreover, it has been shown that distinct stress factors like heavy metals or antibacterial biocides may also contribute to the promotion of various ARGs dispersion via the co-selection phenomenon, as genes conferring resistance to these compounds are frequently co-localized with ARGs within particular MGE (16).
Amongst various natural and anthropogenically-shaped environments, wastewater treatment plants (WWTPs) are considered one of the most common places where microorganisms are subjected to large number of diverse environmental stressors the same moment. This leads to selection of microbial opportunists (including multidrug-resistant bacteria) that can survive exposure to various toxic compounds (17). Emergence of multidrug-resistant strains takes place mostly in WWTPs where sewages from high antibiotics (but also heavy metals and biocides) consumption sectors (e.g. health service and agriculture) mix. Thus, WWTPs are considered point sources of accumulated and diverse emerging pollutants, including antibiotics, antibiotic resistant bacteria and ARGs (18). WWTPs are also the most important interfaces between the human population and the environment (19). Therefore, there is a great need for surveillance of ARGs in WWTPs, which will allow an epidemiological risk assessment of these emerging pollutants in urban environments.

It is also worth mentioning that the occurrence and diversity of ARGs in other environments, including pristine regions (e.g. Arctic permafrost, deep oceans and subsurface waters), is of a great interest as it can give us an insight into evolutionary origin of antibiotic resistance. Such studies become more and more popular nowadays (20-22), thus suitable scientific tools are needed to enable obtaining reliable results that may give us a broader view of this phenomenon in a future.

There are two main methodological approaches to these kind of analyses, either classical (i.e. culture-based) or molecular (i.e. culture-independent). Culture-based methods have an important limitation - they are significantly biased, as we are able to cultivate less than 1% of known bacterial species (23). Molecular-based techniques, like metagenomics or PCR screening of specific genes might be applied to overcome this limitation. PCR screening of environmental samples is a cheap, quick and easy method enabling insight into microbial diversity and occurrence of particular genes in any given environment. Additionally, several modifications of the PCR method, such as qPCR and RT-qPCR, have been developed to enhance its utility (24-25). PCR screening is a common method used for clinical and environmental screening of ARGs distribution and diversity. However, performing reliable PCR screening of ARGs, requires properly selected PCR primers (26). Unfortunately, plethora of possible ARG-specific PCR primers present in scientific literature complicate the selection step significantly. It was shown, that certain primer pairs are genus-specific, which significantly limits their application and may lead to obtaining the false negative results, while other primers show relaxed specificities (i.e. they are specific not only to particular group of ARGs, but also to other genes), which may result in false positive results. With respect to emerging threat linked with an environmental dissemination of antimicrobial resistance, we found that it is crucial to gather this PCR primers and classify them, to enable reliable monitoring of ARGs in various environments, including anthropogenically shaped ones, like WWTPs. Therefore, the aim of this study was to develop a literature-based and curated primers database (LCPDb) designed to facilitate screening of the occurrence and diversity of various ARGs in environmental and clinical samples. Moreover, using prepared by us algorithms, for each of the analyzed primer pairs the specificity, efficacy and taxonomic efficacy parameters were calculated. This allowed preparation of primer pairs rankings that facilitates the user choosing proper primers for the particular application.

*In silico* methods are known according to which the PCR process is not conducted in laboratory environment, but rather reproduced in a computer. Such *in silico* PCR process is also known as ePCR. Likewise, a BLAST procedure *in silico* is known (*basic local alignment search tool*), which is a procedure for comparing primary biological sequence information, such as the amino-acid sequences of proteins or the nucleotides of DNA and/or RNA sequences.

US patent no. US 10100354 B2 and US patent application no. US 2017 0053061 A1 disclose an *in silico* PCR method applied in order to check whether a group of pre-selected primers (starters) would or would not amplify unwanted products or whether the primers would interact among themselves. Such interactions are undesirable in case of so-called Multiplex PCR, in which several primers are used simultaneously in order to obtain several products at the same time in one PCR reaction. Therefore, for the Multiplex-PCR one needs highly selective and specific primers. The aforementioned US patent publications disclose scoring of primers, based on their physical and chemical parameters. In turns, suitable primers are selected based on the scoring. This is a standard, typical procedure, and well known in the art, used for individual cases. Many computational tools are known and available, which implement this procedure *in silico.* In such cases, the reference database of gene sequences is typically narrow - limited to target sequences, i.e. these sequences, which are intended for amplification. To the contrary, the present invention uses as wide gene database as possible as the reference database - by way of example it can be the so-called NCBI database (*National Center of Biotechnology Information* database), comprising the most extended publicly available database of DNA sequences. As the result, the method disclosed in the present patent application allows not only to avoid unwanted interactions between selected primers if used together in Multiplex-PCR, but moreover allows for selection of such primers, which would amplify all the sequences intended for amplification and only such sequences.

Publications are known (Design of Primers for Evaluation of Lactic Acid Bacteria Populations in Complex Biological Samples, Qiangchuan Hou et al., Front Microbiol. 2018; 9: 2045 and Development of a Prokaryotic Universal Primer for Simultaneous Analysis of Bacteria and Archaea Using Next-Generation Sequencing, Shunsuke Takahashi at al., PLoS ONE 9(8): e105592), which disclose methods aiming at selecting "universal" primers, i.e. primers with high efficacy.

The publication entitled Primer-BLAST: A tool to design target-specific primers for polymerase chain reaction, Jian Ye et al., BMC Bioinformatics, 2012; 13: 134) discloses application of in silico PCR for checking in a possibly wide scale what a selected pair of primers amplifies, taking as the reference database - a big database (NCBI). However, the procedure disclosed in this publication ends with a report containing a list of obtained products. There is no suggestion of using the obtained products or assignment of any scoring parameters to the primers (which is the subject of the present invention). Moreover, the NCBI reference database is used as the only reference database according to this publication. To the contrary, the present method proposes constructing additional reference database, for a yet more accurate scoring and selection of primers.

Last but not least, a method similar to the present invention is known from the article entitled In silico assessment of primers for eDNA studies using PrimerTree and application to characterize the biodiversity surrounding the Cuyahoga River, M. V. Cannon et al., Scientific Reports volume 6, Article number: 22908 (2016). In this publication, the results are visualized as a phylogenetic tree. To the contrary, according to present invention, primer pairs are scored using numeric parameters, which is much more convenient in most applications.

The initial concept, on which the currently proposed invention is base, was revealed in post-conference materials after the 15th International Conference on Environmental Science and Technology. The publication entitled Computational prediction, rationalization and experimental validation of PCR primers for the detection of antibiotic resistance genes in wastewater treatment plants, Decewicz P., Gorecki A. et al., (available at https://pdfs.semanticscholar.org/5573/fd6d2df64e992747aa959e9886222b9c18a7.pdf) discloses an idea of using *in silico* PCR against global databases as a reference for testing of PCR-primers, however a detailed explanation of the algorithm used wasn't given. There are also differences in names of parameters, way of calculating parameters and databases which were used for calculations between that disclosure and currently proposed invention.

As far as the authors of the present invention know, the present solution is the first and only one compiling automatically all advantages of the procedures disclosed in the aforementioned publications, based on a reliable, wide reference database of gene sequences and on carefully proposed scoring parameters to describe the performance of primer pairs in question.

### Summary

A method of selecting PCR primers, preferably for detection of antibiotic resistance genes, according to the invention, comprising the following computer-implemented steps:
a) providing a first reference gene sequence database comprising first gene sequences;
b) providing a second reference gene sequence database comprising second gene sequences;
c) searching said first gene sequences in said second reference gene sequence database;
d) creating a third redundant gene sequence database comprising the gene sequences found as the result of step c);
e) creating a third non-redundant gene sequence database by selecting all and only unique gene sequences from said third redundant gene sequence database;
f) providing a test primer database, comprising primer pairs for amplifying gene sequences;
g) performing in silico PCR using as a matrix said second reference gene sequence database and said test primer database;
h) counting the number P of products obtained in the step g) for each primer pair from said test primer database;
i) for each of the P products, searching for it in said third redundant gene sequence database and counting the number of products found therein and correct products CP;
j) performing in silico PCR using as a matrix said third non-redundant gene sequence database and said test primer database;
k) counting the number RP of reference products obtained in the step j) for each primer pair from said test primer database;
l) for each primer pair from said test primer database: calculating the taxonomic diversity of products as the number PT of taxons from which the products of step g) originated and the reference taxonomic diversity as the number RT of taxons corresponding to all variants of a particular gene sequence from said first reference gene sequence database found in said third redundant gene sequence database;
m) for each primer pair from said test primer database:
   calculating the primers specificity parameter S as the ratio of correct products CP to the number P of products obtained in the step g); S=CP/P;
   calculating the primers efficacy parameter E as the ratio of reference products RP to the number NRER of the non-redundant variants of a particular gene in said third non-redundant gene sequence database; E=RP/NRER;
   calculating the primers taxonomic efficacy parameter TE as the ratio of the taxonomic diversity of products PT to the reference taxonomic diversity RT; TE=PT/RT;
n) for each primer pair from said test primer database: assigning parameters of the primers specificity parameter S, the primers efficacy parameter E and the primers taxonomic efficacy parameter TE to said primer pair;
o) selecting primer pairs according to their values of the primers specificity parameter S and/or the primers efficacy parameter E and/or the primers taxonomic efficacy parameter TE.

Preferably, said first reference gene sequence database is the Bacterial Antimicrobial Resistance Reference Gene Database (BARRGDB).

Preferably, said second reference gene sequence database is the National Center for Biotechnology Information Nucleotide Database (NCBI NT).

Preferably, step c) is performed using the BLASTn algorithm, preferably with the threshold of 90% sequence coverage and 90% sequence identity.

Preferably, step e) consists in dereplicating said third redundant gene sequence database, preferably using the VSEARCH tool.

Preferably, step g) is performed using ePCT tool, preferably with the following parameters: . word size: 6, e-value: 10000, reward for positive match: 1, penalty for mismatch: -2, penalty for gap opening: 5, penalty for gap extension: 3, maximum number of aligned sequences to keep: 10000, minimum query coverage per subject: 75.

Preferably, said taxons correspond to bacterial families.

Preferably, step o) primer pairs having higher values of the primers specificity parameter S and/or higher values of the primers efficacy parameter E and/or higher values of the primers taxonomic efficacy parameter TE are selected.

Preferably, said first reference gene sequence database and/or said second reference gene sequence database and/or said test primer database is created based on a biological sample.

Preferably, the primer pair or primer pairs selected is step o) are applied in a laboratory environment, preferably to amplify target gene sequence(s).

### Brief Description of the Drawings

Preferred embodiments of the present invention are presented in a more detailed way with reference to the attached drawing, in which:
Fig. 1 presents the summary of antibiotic resistance genes for which at least one PCR primer pair is available in the CPDb. and
Fig. 2 shows flowchart showing algorithm implemented for obtaining the specificity (S), efficacy (E) and taxonomic efficacy (TE) parameters of the PCR primers collected in the LCPDb, and
Figs. 3a, 3b present *in silico* comparison of the efficiency of ARGs screening using lowest- and highest-ranked primer pairs (available in the LCPDb) tested against various metagenomes of pristine and anthropogenically-shaped environments, and
Fig.4 shows detection of ARGs (for which primers are available in CPDb) in various environmental metagenome datasets using an "in silico" amplification by ePCR tool. Concentric circles represent environments, green color presence of ARG detected by at least one variant of primer pairs (available in CPDb) designed to analyzed ARG, and
Fig.5 presents comparision of ARGs screening using selected lowest- and highest-ranked primer pairs (available in the LCPDb) detected by conventional PCR in total DNA isolted from primary sludge from WWTP in Oswiecim Poland;
Fig. 6 shows correlation plot between all CPDb parameters.

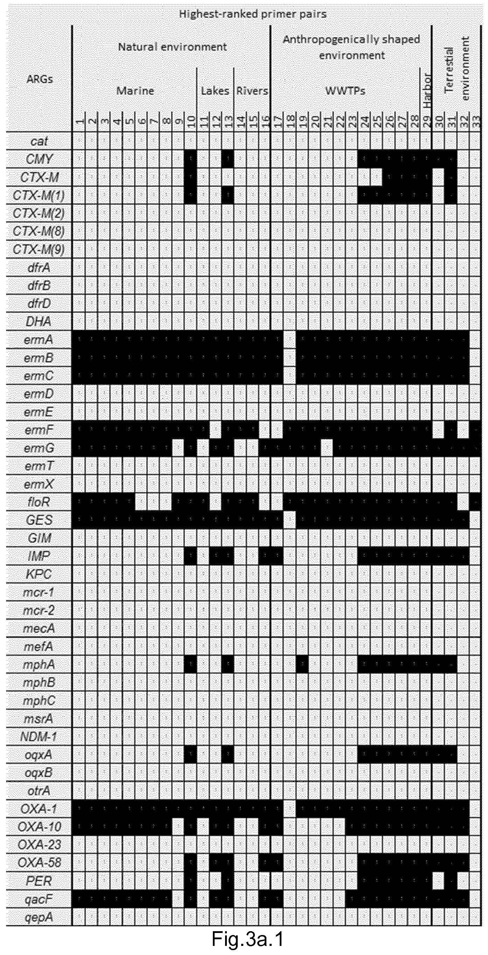

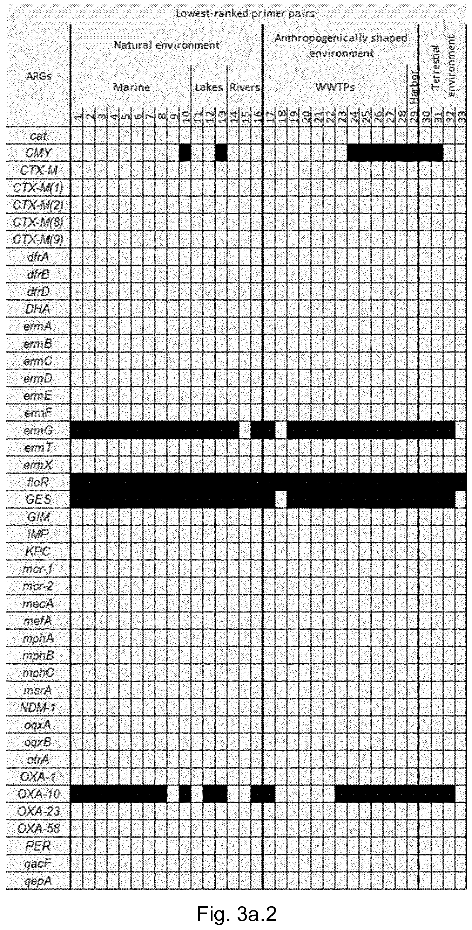

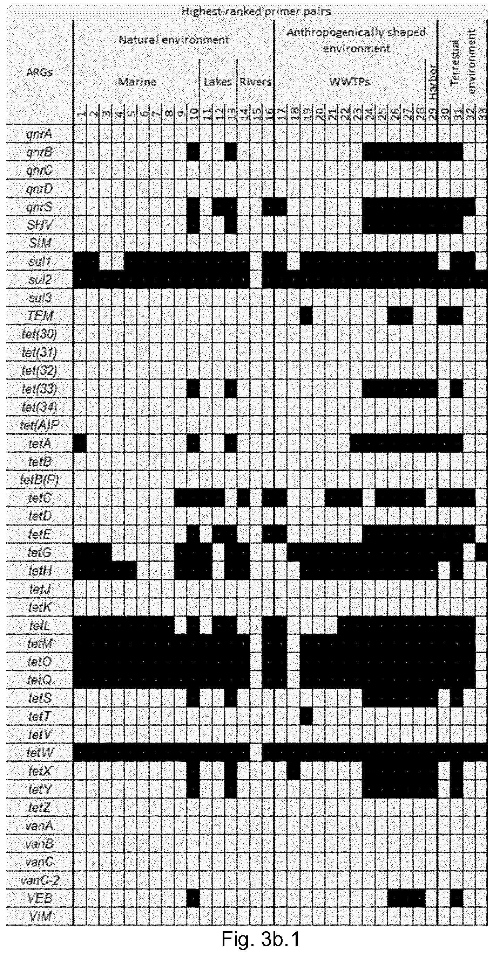

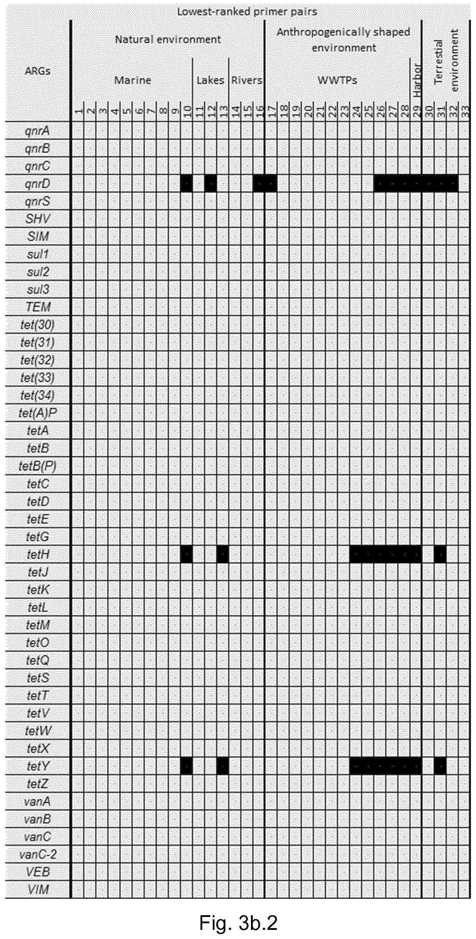

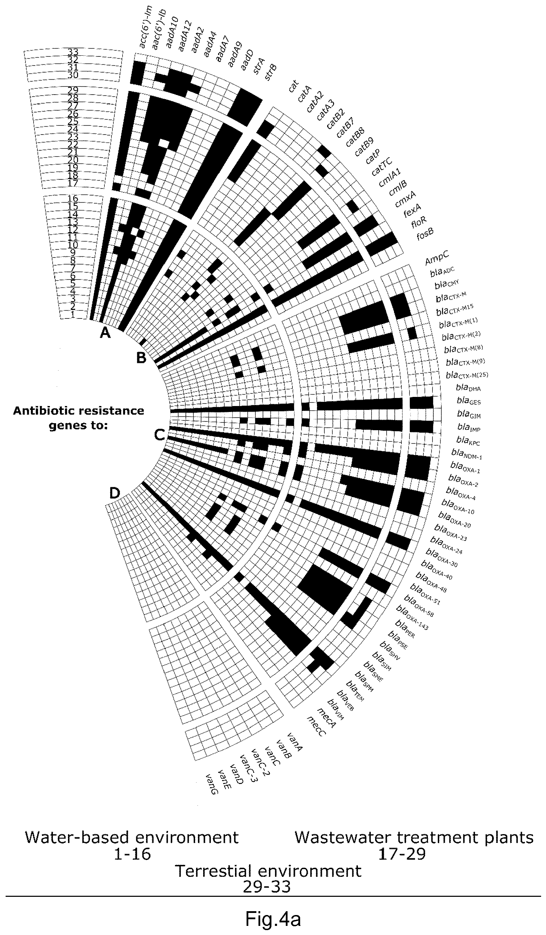

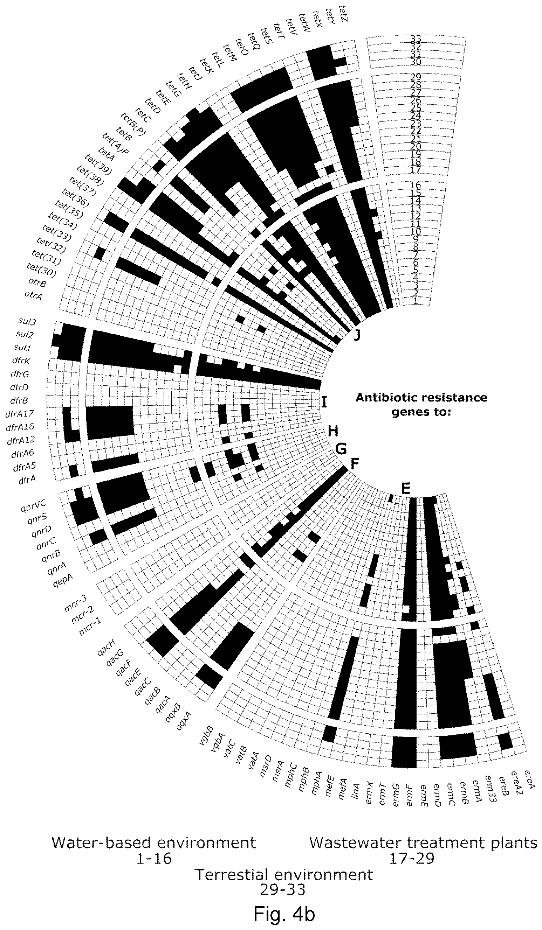

In Figs. 3a, 3b, and 4, there were used following abbreviations: 1 - Vaz Islands (Brasil); 2 - Mediterranean Sea (Malta); 3 - Atlantic Ocean (N/A); 4 - Agulhas Bank (RPA); 5 - Gulf of Oman (Oman/Iran); 6 - Indian Ocean (India); 7 - Eurpa Island (France); 8 - Pacific Ocean (N/A); 9 - Arctic Ocean (N/A); 10 - South China Sea (N/A); 11 - Meyghan (Iran); 12 - Rabindra Sarovar (India); 13 - Carter (Mexico); 14 - Xiangxi (China); 15 - Surat (India); 16 - Salmon Plume (Columbia); 17 - Aarhus (Denmark); 18 - Hachioji (Tokyo); 19 - Cambridge (United Kingdom); 20 - Stockholm/Gashaga (Sweden); 21 - Stockholm/Hennriksadals (Sweden); 22 - Sheffiled (United Kingdom); 23 - Southampton (United Kingdom); 24 - Vermont (United States); 25 - Odense (Denmark); 26 - Shanghai (China); 27 - Shifflange (Luxemburg); 28 - Hong Kong (China); 29 - Singapore (Singapore); 30 - Jaxing (China); 31 - Gulf of Cadiz (Spain); 32 - Kamloops (Canada); 33 - Healy (United States); A-Aminoglycosides; B - Amphenicols ; C - β-lactams; D - Glycopeptides; E - MLS - group; F - Multidrug resistant pumps; G - Polymixins; H - Quinolones; I - Sulfonamides; J - Tetracyclin;

Table 1 shows list of primer pairs used for PCR, and
Table 2 presents list of metagenome used for *in silico* analysis,

### Detailed Description

### 1. Preferred embodiments

Preferred embodiments of the invention are described in details below. The examples serve only as an illustration and do not limit the scope of the present invention.

### 2. Materials and methods

### 2.1. Samples collection

The samples were collected from the municipal and industrial WWTP located in Oswiecim (Poland) in November 2014. The WWTP has the capacity of about 53 400 m3 waste per day.

### 2.2. DNA extraction

The total DNA was extracted from 100 mg of frozen sludge samples using Fast DNA Spin Kit for Feces (MP Biomedicals, Illkirch, France) according to the manufacturer instructions. The DNA concentrations and purity were determined using Qubit™ 2.0 Fluorometer (Invitrogen, Carlsbad, CA, USA).

### 2.3. Antibiotic resistance genes screening in WWTP from Oswiecim

Occurrence of antibiotic resistance genes in the analyzed samples was investigated using PCR assays. The PCRs were performed with 10 pmol of each primer (Table 1), DreamTaq PCR Master Mix (Thermo Fisher Scientific, Waltham, MA, USA) and 150 ng of matrix DNA. The PCR programs were dependent on recommended annealing temperature for each primer pair (Table 1). The PCR products were resolved by electrophoresis in an 2% agarose gels.

### 2.4. Standard molecular biology techniques

The PCR products were cloned into pTZ57R/T vector (Thermo Fisher Scientific, Waltham, MA, USA) and introduced via chemical transformation into *E. coli* DH5α (27). Plasmid DNA was purified using Plasmid Miniprep DNA Purification Kit (EURx, Gdansk, Poland) and sequenced using universal primers (M13/pUC) and capillary DNA analyzer ABI-PRISM 377 (Applied Biosystem, Foster City, CA, USA) at the Institute of Biochemistry and Biophysics, Polish Academy of Science (oligo.pl).

### 2.5. ePCR tool

To test primer pairs, ePCR tool was developed in Python v3.4 which wraps nucleotide BLAST program (28) to search primers against selected databases or fasta files. BLASTn parameters were optimized for short query sequences i.e. word size: 6, e-value: 10000, reward for positive match: 1, penalty for mismatch: -2, penalty for gap opening: 5, penalty for gap extension: 3, maximum number of retained HSPs (High Scoring Segments per subject sequence): 10000, minimum query coverage per subject: 75.

### Throughout the present patent application:

**BLAST** stands for Basic Local Alignment Search Tool, which is a suite of programs for comparison of DNA or protein sequences. In its foundations it implements heuristic algorithm of Smith-Waterman algorithm for local comparison of sequences, while providing highly accurate, although not always optimal, alignments of compared sequences with much lower computational and time cost. One of the program from that suite is **BLASTn,** which is a program dedicated the comparison of nucleotide sequences. This programs splits any query sequence (like primer) into smaller parts called k-mers, where k corresponds to the size of that part. These k-mers are also called or referred to as **words** or subwords of the query and thus these are controlled by the word_size parameter (**word size: 6** means a k-mer having the size of k=6). These k-mers are further compared with the database of k-mers acquired from the reference sequences deposited in a specially formatted database, acquired by a different program from BLAST suite, called makeblastd. During the comparison of k-mers and their extension to longer alignments or **High Scoring Segments** (**HSPs**, controlled by max_hsps parameter) k-mers are scored based on the identity of aligned nucleotides in the following manner: when two nucleotide match (e.g. A to A) a reward of 1 point is assigned **(reward for positive match:** 1), if a mismatch occurs (e.g. A to G), 2 points are subtracted (**penalty for mismatch:** -2). The sum of points is the score of the whole alignment. However, when a gap occurs in the alignment (e.g. AA-GC to AATGC) there is a penalty of 5 points (in our setting) (**penalty for gap opening:** 5) and if it is extended (e.g. AA--C to AATGC) another 3 points are subtracted from the alignment (**penalty for gap extension:** 3). Another parameter limiting the number of obtained alignments in the further analysis of primers parameters is ***e-value*** score. It is a statistic calculated from the compared sequences and obtained alignments informing about the probability of observing the obtained alignments in a random way, hence the lower the value the lower the probability of random alignment. However, this parameter is strongly influenced by lengths of query and subject sequences, e.g. while comparing short sequence, like a primer of average length of 20 nucleotides, and a chromosome sequence od 4 000 000 nucleotides the e-value rises dramatically. To allow the alignments to have very high e-value is against of its fundamental purpose but it's non-trivial usage is of a great usefulness in the case of testing every possible location of a short primer sequence. Then, the obtained alignments are checked whether the **minimum query coverage** condition was met. This parameter requires the query sequence to align to the subject sequence, against which is it compared to, to cover at least 75% of its length, e.g. when the query is 20 nt-long, 15 nucleotides has to align and create a single HSP to be included in further analysis. Lastly only the maximum of **HSPs** obtained from the sequence against which the search was performed is retained. The BLASTn program is envoked from within the Python script in which the algorithm for primers parametrization was implemented.

### 2.8 Analysis of various environmental metagenomes datasets

The validation of all the analyzed primer pairs were conducted with the use of the following environmental metagenomes datasets downloaded from the EBI metagenomics database (29): ERR594355 from Trindade and Martin Vaz Islands (Brasil), ERR594358 from Mediterranean Sea (Malta), ERR594388 from Atlantic Ocean (N/A), ERR594389 from Agulhas Bank (RPA), ERR594402 from Gulf of Oman (Oman/Iran), ERR594403 from Indian Ocean (India), ERR599339 from Europa Island (France), ERR599368 from Pacific Ocean (N/A), ERR1855270 from Arctic Ocean (N/A), SRR2135746 from South China Sea (N/A), ERR1739733 from Meyghan Lake (Iran), ERR739056 from Rabindra Sarovar Lake (India), SRR3311153 from Carter Lake (Mexico), ERR1474558 from Xiangxi (China), ERR1951254 from Surat (India), ERR864073 from Salmon Plume (Columbia), ERR712390 from Aarhus (Denmark), DRR066657 from Hachioji (Japan), ERR1193298 from Cambridge (United Kingdom), ERR1414223 from Stockholm/Gashaga (Sweden), ERR1414223 from Stockholm/Hennriksadals (Sweden), ERR1981052 from Sheffiled (United Kingdom), ERR1981059 from Southampton (United Kingdom), SRR866802 from Vermont (United States), SRR999554 from Odense (Denmark), SRR1144841 from Shanghai (China), SRR1611146 from Shifflange (Luxemburg), SRR2043638 from Hong Kong (China), SRR2135767 from Singapore (Singapore), SRR1029957 from Jaxing (China), SRR1237971 from Gulf of Cadiz (Spain), ERR753916 from Kamloops (Canada), ERR1039458 from Healy (United States). Abovementioned metagenomes were assembled using metaSPAdes v3.11.1 (30). Obtained contigs were afterwards searched with ePCR tool to check for the presence of selected ARGs

### 3. Results and discussion

### 3.1. Construction and overall structure of the Literature-based Curated Primers Database (LCPDb)

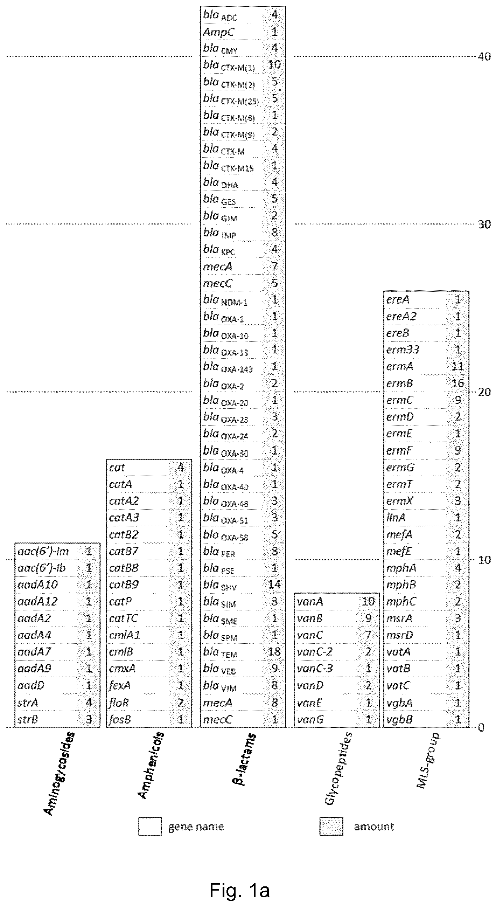

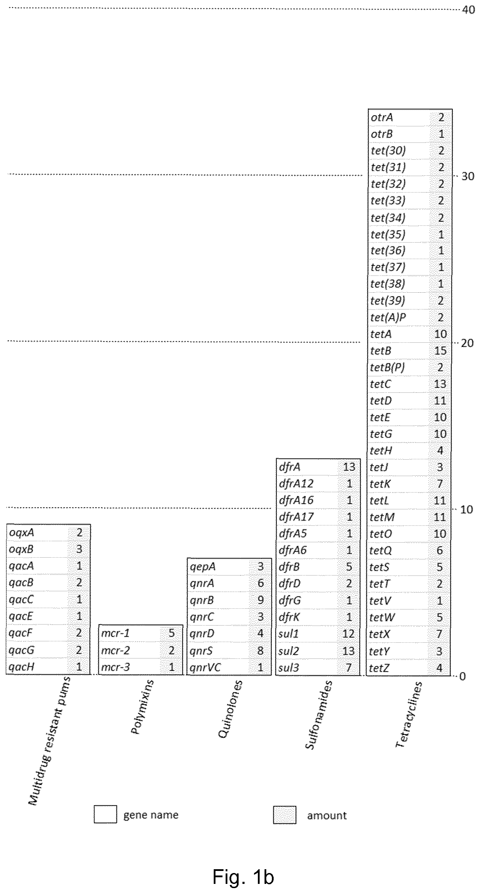

The aim of this study was to create a database of PCR primers enabling rapid and reliable detection of antibiotic resistance genes in various environmental and clinical samples. It was developed based on the thorough scientific literature review followed by manual verification of each primer pair. The Literature-based and Curated Primers Database (LCPDb) is available under the following link: cpdb.ddq.biol.uw.edu.pl. Currently, the database contains 607 primer pairs, that were designed for amplification of 169 various ARGs conferring resistance to various classes of antibiotics, including: (i) aminoglycosides (16 primer pairs), (ii) amphenicols (20), (iii) β-lactams (171), (iv) glycopeptides (33), (v) macrolides, lincosamides, streptogramines (MLS)-group (80), (vi) polymixins (8), (vii) quinolones (34), (viii) sulfonamides (59), (ix) tetracyclines (171) and (x) 15 primer pairs designed for amplification of genes encoding multidrug resistance pumps (Fig. 1).

Currently LCPDb has been supplemented with information about reference of deposited primers (if one was available). It is noted by PMID number (PubMed Identifier). Additionally few basic parameters of primer pairs were added, namely primer length, GC-content and melting temperature for various DNA polymerases. However in the future we plan to extend the database with additional parameters, such as whether a primer pair may be used in qPCR analysis.

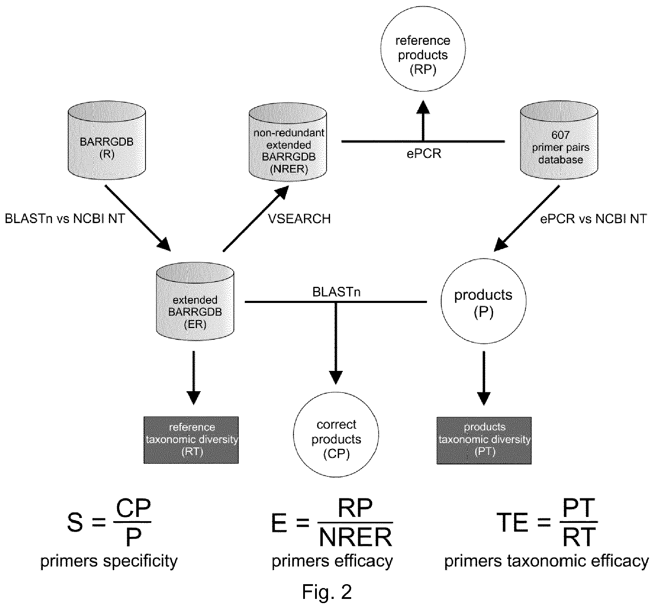

Furthermore, the database was extended with internal ranking of the PCR primer pairs based on three parameters, i.e. specificity (S), efficacy (E), and taxonomic efficacy (TE) (Fig. 2). For estimating of the abovementioned parameters of the primer pairs collected during the data mining a series of *in silico* PCR (performed applying designed in this study ePCR tool) was performed using as a matrix DNA the resources of the NCBI nucleotide NT database and the Bacterial Antimicrobial Resistance Reference Gene Database (BARRGDB) (31-32) (Fig. 2). Obtained parameters allow the user appropriate selection of reliable primer pairs upon her/his needs.

For the analysis of 607 primer pairs included in the LCPDb **[the test primer database]** at first the Bacterial Antimicrobial Resistance Reference Gene Database (BARRGDB) (32) **[the first reference gene sequence database]** was used, and then it was extended by blasting extracted ARGs against the NCBI nucleotide (NCBI NT) database (34) **[the second reference gene sequence database]** with a threshold of 90% sequence coverage and 90% sequence identity. Throughout the present patent application these mean that each sequence has to align to each other over 90% of their lengths **(90% sequence coverage)** and the aligned regions has to show at least 90% identity in total **(90% sequence identity).** This allowed identification of the variety of ARGs present in the NCBI NT and resulted in obtaining the extended BARRGDB, named the ER database **[the third redundant gene sequence database].** Next, *in silico* PCR (with the usage of the ePCR tool) was performed using as a matrix the NCBI NT resources and 607 primer pairs, and the obtained DNA products (P) were compared with the ER database using the BLASTN (with the following threshold: 90% sequence identity and 90% query coverage) to indicate the set of the correct products (CP). This was used to calculate primer pairs specificity (S) parameter as the ratio of the number of correct products (CP) to all possible products (P). Then, resources of the non-redundant variant of the ER database (NRER) **[the third non-redundant gene sequence database]** (obtained using the VSEARCH tool (35) were used as a matrix for *in silico* PCR applying all tested primer pairs. To avoid any doubts regarding functionality of the VSEARCH tool it is explained that what the VSEARCH tool does is - it dereplicates the sequences from the ER database (or - in other words - it retains all unique sequences from the ER database). In turns, it can also be said that the resources of the non-redundant variant of the ER database (NRER) obtained by retaining all unique sequences from the ER database, were used as a matrix for in silico PCR applying all tested primer pairs. Or, that the resources of the non-redundant variant of the ER database (NRER) obtained by dereplicating sequences from the ER database, were used as a matrix for in silico PCR applying all tested primer pairs. As the result the number of variants for each ARG covered by particular primer pair was calculated, i.e. reference products number (RP). Based on these calculations, the efficacy (E) parameter could be estimated, as the ratio of the reference products number (RP) to the number of the non-redundant variant of particular gene in the NRER database. Finally, to obtain the taxonomic efficacy (TE) parameter for each primer pair the ratio of the number of taxons for which the PCR products P (taxonomic diversity of products - PT) were assigned to the number of taxons that all variants of particular ARG found in the ER database (taxonomic diversity of variants; reference taxonomic diversity - RT) was calculated. In each case the taxonomic diversity of particular DNA sequence (*in silico* PCR product or ARG variant) was based on taxonomy assigned to the bacterial host.

### 3.1.1 The specificity (S) parameter

Antibiotic resistance genes have been identified as global threat to public health and since then the increased attention to occurrence, diversity and spread of ARGs in various environmental compartments has been drawn (33). The antibiotic resistance is mainly monitored applying variations of polymerase chain reaction method (PCR), which all need to be preceded by designing specific (to appropriate ARGs) oligonucleotide primers. After several years of applying PCR for such screening of the ARGs, hundreds of primer pairs have been described in scientific literature. We performed a global analysis of these primers and we found that some of them show relaxed or even lack of any specificity to the intended target gene. Therefore, we decided to include in the CPDb the specificity parameter that was calculated for each primer pair. It shows the probability that the obtained DNA product represent a variant of a desired target gene and not a random DNA fragment.

That parameter takes values from 0 to 1, where 0 means possibly lowest specificity and 1 reflects the highest specificity. We found that 99 out of 607 analyzed primers exhibited specificity value equal to 0. It shows that there are a lot of mistakes in already published manuscripts. As we found after thorough investigations in most cases, low value is a result of incorrectly annotated gene name of particular ARG (e.g. some primers specific to the *tetA* gene are in fact specific to the *tetC* gene). Encountered situation is also incorrectly assigned (or even missing) estimated size of the PCR product (26/607 primer
pairs). The amplicon size is crucial for determining whether obtained products correspond to the gene of interest and can be further analysed. The ePCR tool used for estimation of the S rate can verify if amplicon size given in literature is correct, otherwise it returns "wrong product size" (W/PS) note to CPDb. The rest of primer pairs 484/607 declare values of the S parameter between 0.04 to 1.

It is worth noting that ARGs are a rapidly evolving group of genes, which results of a great diversity of genes within each family. However, as these genes very often have a common ancestor they also share highly conserved DNA regions that are frequently used for the PCR primer designing. Such approach significantly lowers the "differentiation power" of particular primers, as they become specific to the group of of ARGs (e.g. *bla*) and not a specific type of ARGs (e.g. *bla*_{TEM}). A good example here is quinolone resistance proteins (*Qnr*). These proteins are pentapeptides repeat proteins that mimic DNA (36). It these groups we distinguish several types like *qnrA*, *qnrB*, *qnrC* etc. Within these types a nucleotide sequence of a gene in particular locus has even 70% of identity. That situation occur e.g. between nucleotide sequence in *qnrB* and *qnrD*. So in that case designing primers within that similar region of genes will drastically reduce specificity of a primer pairs.(37)

Therefore, based on our observation, we recommend the LCPDb users to apply primers with possibly highest value of the S parameter, especially with rates higher than 0.95 (that was calculated for 435/607 of primer pairs).

### 3.1.2 The efficacy (E) parameter

ARGs pose a point of interest from early 1989's when the first nucleotide sequence of the *ampC* gene was described (46). Since then, more and more scientific groups have tried to reveal what genes are responsible for the particular resistant phenotypes. When PCR method was introduced in 1983 (38) rapid identification of ARGs in nosocomial and non-nosocomial bacteria became possible. Back then, scientists have used individual nucleotide sequences of ARGs as a template to design primes for PCR, which may strongly influence of the results of their future analyses. Twenty years later, when next-generation sequencing methods were introduced, thousands of various variants of the ARGs were revealed (39-40). This allowed designing primers on the basis of conserved regions of multi-aligned ARGs which significantly increased chances of amplifying more variants of specific ARG, and reducing the PCR bias.

In the LCPDb, the efficacy (E) parameter was calculated for every PCR primer to fill this gap and provide the percentage of covered homologs of specific ARGs. The efficacy (E) parameter, was calculated as a ratio of all good *in silico* products to all homologs identified in the NT database (Fig. 2). The E parameter takes values between 0 and 1 and may varies significantly between different primer pairs designed for amplification of particular ARGs, e.g. in the LCPDb 5 primer pairs for the *qnrS* gene have E values between 0.26 to 1.

It is worth mentioning that during the analysis we observed primer pairs for which even though the value of the S parameter was high, the efficacy value equaled 0. That case was observable when expected amlicon size of target gene was at least 10% longer than complete gene sequence. It is typically occur when researchers are willing to obtain functional ARGS for further downstream applications thereby it is necessary to use primer pair designed based on the nearest intergenic region of the ARG (41-42). In these cases (18/607 primer pairs) the values for the E parameter in the LCPDb were changed from 0 to the note "bind outside the gene".

For the purpose of the accurate ARGs screening in various environments we recommend choosing primer pairs with possibly highest efficacy values or the note "bind outside the gene" (if other parameters are satisfying). This enables to reduce significantly the chances of obtaining false negative results.

### 3.1.3 The taxonomic efficacy (TE) parameter

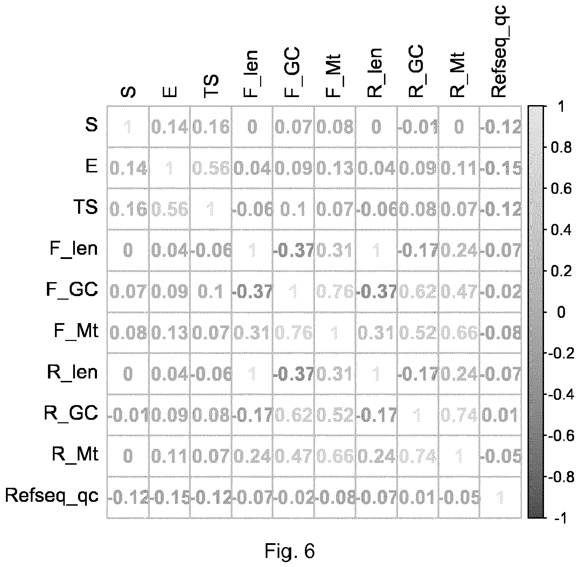

Environmental conditions have relevant influence on a structure of microbial community and the distribution of bacterial taxa between various environments shows considerable differences (43-44). On the other hand, taxon-specific genomic features (like distinct codon usage or GC-content) strongly influence the efficacy of the PCR screening of genes of interest using particular primer pairs (45). Accordingly, the taxon efficacy (TE) parameter has been added to the LCPDb. The TE is a derivate of the efficacy parameter and shows the user within how many taxa, specific ARGs may be amplified using the particular primer pair. Taxonomic efficacy (TE) was calculated as the ratio of the number of taxons for which the PCR products obtained applying particular primer pair were assigned to the number of taxons in which all variants of the particular ARG were found in the extended Bacterial Antimicrobial Resistance Reference Gene Database (32). The TE and E parameters are positively correlated (Fig. 6), but in case of TE, values range from 0 to 1. Higher TE value indicates that a primer pair can detect given ARG in more bacterial families. Additionally, the LCPDb is equipped with an option to list the taxa covered by particular primer pair. It might be useful for establishing expectations of an experimental design (i.e. whether one is looking for ARGs related with specific strain/strains or want to conduct general screening of ARGs in the particular environment).

As mentioned before, some variants of ARGs that are found in taxonomically distinct strains, may significantly vary in their nucleotide sequences (47). In these cases it is often impossible to design universal primer pairs to cover all taxonomic groups of bacteria encoding specific ARGs. For such cases it is required to use more than one, complementary working, primer pair to cover the possible pool of hosts of particular ARG. Revealing the taxonomic diversity of the particular environment is currently a standard procedure applying sequencing of the 16S rRNA gene as a part of metataxonomic approach or regular shotgun metagenomic sequencing of the sample. Additionally, currently there are terabytes of sequencing data from various environments available online in public databases (e.g. SRA and ENA databases) many of which have taxonomy details easily accessible through web services (e.g. MG-RAST, ENA or MGnify). This data may be used as a reference for an appropriate experimental design, including selecting reliable primer pairs. Thus, prior to PCR screening of ARGs, we highly recommend at least checking taxonomic diversity in similar environments or using primer pairs covering broad range of taxa.

### 3.2. Analysis of the distribution of ARGs in metagenomes of anthropogenically-shaped and pristine environments - in silico testing of the LCPDb application

To test the utility of the parameters included into the LCPDb in proper designing of the experimental setup an *in silico* analysis was conducted using 33 various metagenomes (available in EBI Metagenomic database [https://www.ebi.ac.uk/metagenomics/]) from water (13), and terrestrial (4) environments, as well as astewater treatment plants (16) (Tab. 2). These environments represents, both i.e. anthropogenically-shaped and primal (only slightly or non-influenced by human) environments. Assembled metagenomes were screened with the usage of a developed ePCR tool for the presence of ARGs. For the screening selected PCR primer pairs available in LCPDb were used. These allowed the comparison of the utility between lowest- and highest-ranked primers (Figs. 3a, 3b) and revealed the overall occurrence of ARGs in various metagenomes (Fig. 4).

The comparison of the efficiency of the lowest- and highest-ranked PCR primer pairs in ARGs amplification allowed validation whether parameters calculated for each primer pair may be used as a reliable criteria for selecting of the most appropriate primers for the PCR screening of ARGs. Given that, Figs. 3a and 3b were compiled using these genes for which at least two primer pairs are available in LCPDb. Then, 88 primer pairs referring to particular ARGs that showed lowest and highest values of S, E and TE parameters were selected. The ePCR tool was used to perform an *in silico* amplification of ARGs using selected (88) primer pairs on 33 environmental metagenome datasets as a matrixes. Therefore, in total, 2904 screening experiments (*in silico* PCR analyses) were performed. En each case the obtained PCR products were verified manually by BLAST testing. Based on this analysis it was shown that highest-ranked primers allowed detection of 41 ARGs in 33 metagenomes, i.e. in total 755 (26%) screening analyses were positive. In contrast, lowest-ranked primers enabled detection of only 8 ARGs in 33 metagenomes, which means that in total only 156 (5.37%) screening analyses revealed to be positive. Obtained result indicated that primers with higher rates (according the LCPDb internal ranking) are nearly five times more effective and confirms the thesis that for planning the reliable analysis only the primer pairs with possibly highest rates should be chosen.

Interestingly, *in silico* PCR amplification of the *ermG* and *floR* genes yielded in obtaining of unexpected results, as the lowest-ranked primer pairs detected ARGs in more environments than the highest-ranked primer pairs. Furthermore, the highest-ranked primer pair for the *ermG* gene resulted in identification of this ARG in different environments than the lowest-ranked primer pair. These phenomena can be explained by the closer analysis of the taxon efficacy of analysed primer pairs, showing which taxa are covered by particular primer pairs. The *ermG* and *floR* genes are good examples of genes whose nucleotide sequences may significantly differ in various taxa, which excludes the possibility of designing one general and effective primer pair for their screening. This implies also the requirement of using a broader set of primer pairs covering various taxa-specific genes to improve the screening of these particular ARGs in environmental samples.
As a supplementation to abovementioned analysis, we investigated the overall occurrence of ARGs in all analyzed metagenomic datasets. For this purpose, all primer pairs available in the LCPDb were used for *in silico* PCR using as matrixes 33 metagenomic datasets (Fig. 4). All obtained products were manually verified.

Performed analysis revealed ARGs that are prevalent in tested environments, i.e. *strA, strB, floR, ermF, sul2, tet(39), tetG, tetW, tetX.* These genes were detected in all analyzed metagenomes. Moreover, it was shown that antimicrobial gene profiles for particular environments are quite similar. Only few unique profiles were obtained, including these for environments no. 10; 13 (water-based), no. 18 (WWTPs) and no. 33 (terrestrial).

Interestingly, even though many articles emphasize that WWTPs are "hotspots" of ARGs (48-49) this analysis showed only slightly higher diversity of ARGs in WWTPs (27,47%) in comparison with results obtained for water-based (21,96%) and terrestrial (24,71%) environments. It suggest that natural environments has similar antimicrobial profile in the meaning of presence of particular type of considered emerging pollutant. However it must be added that carried out analysis is based on diversity (qualitative) differences. Deduced in published articles conclusion considered WWTPs as "hotspots" based mainly on quantitative approaches (50-51). The facilities responsible for biological sewage treatment process creates an niches potentially suitable for antimicrobial resistance development. Abundance of various antibiotic resistance bacteria (ARB), sub-inhibitory concentration of antibiotics strongly promote mobilization and replication of genes conferring resistance to antibiotics. Consequently increase overall pool of ARGs in these types of environments. Another fact is that natural environments not always equal pristine environments. Movements of water bodies (52-54), zoonotic transmission (55) and release final effluents to rivers (56-57) have massive impact on spread of ARGs in natural environments. An additional matter that needs to be considered during making conclusions from obtained results (and also from all metagenomic-based approaches) is the fact that analyses have been done based on nucleotide sequences generated by various sequencing platforms (e.g. Illumina Miseq, Ion Torrent). Although chosen metagenomic datasets were filtered to select only those that are after deep sequencing (Tab. 2), these will not reflect the full diversity of the examined environment. It is well known that the highest possible saturation obtained for sequencing fluctuate around 80% of overall genetic information present in particular environment (58)

An interesting antimicrobial gene profile was obtained for the permafrost metagenome (ERR1039458; Healy, USA) where only 11 out of 167 tested ARGs were detected. From all selected metagenomic datasets this one, based on physico-chemical conditions, reflects the most pristine environment. In this metagenome only the genes conferring resistances to aminoglycosides (*strA*, *strB*), amphenicols (*catB7*, *florR*), MLS-group (*ermF*, *ermG*), sulfonamides (*sul2*) and tetracyclines (*tet(39), tetG, tetW, tetX*). With respect to emerging threat linked with an environmental spread of antimicrobial resistance it is crucial to focus not only on surveillance of occurrence, diversity and spread of ARGs in anthropogenically-shaped biocenoses, but to extend analyses for pristine environments (e. g. sub-polar permafrost, deep Arctic and Antarctic regions), which are significantly understudied. Deep and accurate analyses of such environments may shed a light on evolutionary bases of ARGs stirring (59).

### 3.4. Analysis of the occurrence of antibiotics resistance genes in the wastewater treatment plant in Oswiecim (Poland) - an experimental testing of the LCPDb application

Recently, regard to expanding problem concern antimicrobial resistance, more and more research groups lead their experiment for an effective monitoring of occurrence, spread and diversity of antibiotic resistance genes (60-62)To achieve that most of scientist use different types of PCR approach, one of most popular is conventional PCR (63-65), which rapidly allows to check whether particular resistance gene is present in examined probes. All procedures comes to run PCR reaction with specific to ARGs primer pair, check if amplified fragment migrated on the expected height by gel electrophoresis and finally analysis of sequence obtained after sequencing of previously prepared DNA piece. Crucial step in this procedure is choosing the properly selected primer pair. To our knowledge, selection step for many if not most, scientist based on a literature review. Ones looking for information of previously designed by authors primer pairs. Usually the information about target gene name, sequences of primer pair and expected size of PCR product may be found in articles. Vast analysis of primer pairs published in articles showed that rely only on these information may in some cases leads to biased results and incorrect conclusion, therefore it is crucial to carry out an additional analysis of PCR primers before we start experiment. Proposed solution for that problem might be LCPDb.

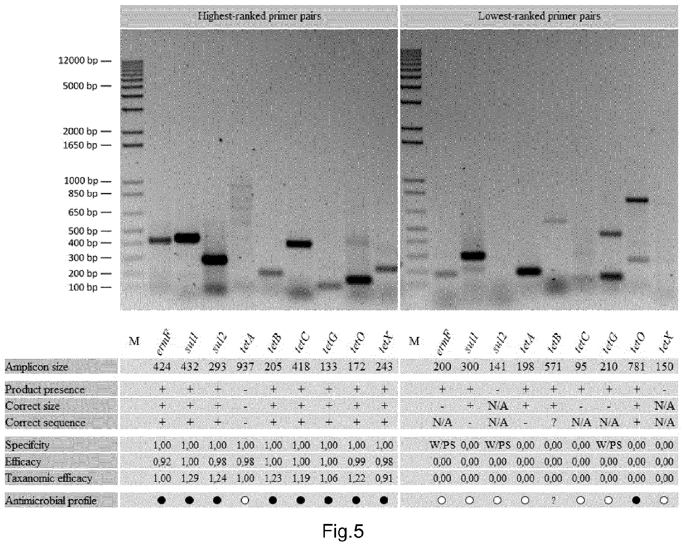

An experimental approach were conducted to check usefulness and correctness of information collected in LCPDb. 18 primer pairs designed to amplify genes conferring resistance to MLS-group (1 primer pair [*ermF*]), sulfonamides (2 [*sul*]), tetracyclines (6 [*tet*]) were selected from LCPDb. Primer pairs were divided by ranking (E, S, TE rates) for 2 groups: highest-ranked primer pairs and lowest-ranked primer pairs. All primer pairs in highest-ranked group declare rates higher than 0.92 whereas primer pairs in lowest ranked group possibly lowest rates founded in LCPDb (Fig. 5). Afterwards standard PCR screening of selected ARGs was conducted to reveal antimicrobial profile of sample collected from first purification process of wastewater (primary sludge) in Oswiecim (Poland).

Results show that antimicrobial profiles obtained by two groups of primer pairs (lowest- and highest-ranked) differ significantly. Highest-ranked primer pairs amplified 8 out of 9 analyzed ARGs (*ermF, sul1, sul2, tetB, tetC, tetG, tetO, tetX*). In the other hand lowest-ranked primer pairs indicate presence of only 1 out of 9 analyzed ARGs (*tetO*).

Gel electrophoresis is a first step to analyze, obtained after PCR reaction results. At that stage we check wheatear we successfully amplified product as well as check if PCR product migrate on the height described in primer pair notification. If all of these conditions have been met, further analysis may be conducted which based on cloning, sequencing and bioinformatics analyses of obtained sequencing results. In case of lowest-ranked primer pairs results show that in two cases there is lack of any product (*sul2, tetX*). Next situation that occurred is inappropriate migration of 3 amplified products (*ermF, tetC, tetG*). It those cases further analysis were discontinue as wrong height of product suggest amplification of unspecific products. In 2 cases correct-size products were rejected after sequence analysis (*sul1*, *tetA*). In those cases sequencing results revealed that amplified products are in fact genes coding multidrug efflux pump and tetracycline efflux pump belongs to C family. It is clearly show that primer pairs with low rates first of all generate biased results (false positives [*sul1, tetA*] and false negatives [*sul2, tetX*]) and secondly unnecessary cost related with cloning and sequencing of unspecific PCR products.

Performed analyses clearly demonstrated that for the studies of the prevalence of antibiotic resistance genes in various environments and their epidemiological risk assessment, only PCR primers with highest possible E, S and TE rates should be used, as only for those, one can be sure that the performed analysis yields correct and high quality results.

### 4. Conclusion

The conclusions drawn from the present work are summarized as follows:
- Antimicrobial resistance caused by antibiotic resistance genes is one of the biggest health threats that mankind faces now and will face in the coming decades. PCR screening with properly designed primer pairs is one of the most conventional approach allows epidemiological risk assessment of emerging pollutants (ARGs) in WWTPs and other environments.
- Additional validation of literature-based primer pairs is crucial to obtain reliable results. Internal ranking of the literature-based PCR primer pairs based on three devised parameters, i.e. specificity (S), efficacy (E), and taxonomic efficacy (TE) significantly reduce chance to obtain biased results.
- Constructed database (LCPDb) is a user-friendly web application which gather information about primer pairs useful in detection of antibiotic resistance genes with rates. LCPDb in easy and quick way allows to choose first-rate PCR primer pairs most useful in particular experiment assumption.
- In the future it is planned to extend database with physico-chemical parameter of primer pairs. Additionally a module with possibility to check rates of self-designed primer pairs by users will be added.

### 5. Bibliography

1. Ventola, C.L., 2015. The antibiotic resistance crisis: part 1: causes and threats. P. T. 40 277-83.
2. Bérdy, J., 2012. Thoughts and facts about antibiotics: Where we are now and where we are heading. J. Antibiot. (Tokyo). 65 441.
3. Piddock, L.J., 2015. Teixobactin, the first of a new class of antibiotics discovered by iChip technology? J. Antimicrob. Chemother. 70 2679-80.
4. Davies, J., Davies, D., 2010. Origins and evolution of antibiotic resistance. Microbiol. Mol. Biol. Rev. 74 417-33.
5. Cromwell, G.L., 2002. Why and how antibiotics are used in swine production. Anim. Biotechnol. 13 7-27.
6. Lipsitch, M., Singer, R.S., Levin, B.R., 2002. Antibiotics in agriculture: when is it time to close the barn door? Proc. Natl. Acad. Sci. U. S. A. 99 5752-4.
7. Arnold, S.R., Straus, S.E., 2005. Interventions to improve antibiotic prescribing practices in ambulatory care. Cochrane. Database. Syst. Rev. CD003539.
8. Wayne, A., McCarthy, R., Lindenmayer, J., 2011. Therapeutic antibiotic use patterns in dogs: observations from a veterinary teaching hospital. J. Small. Anim. Pract. 52 310-8.
9. Pruden, A., Pei, R., Storteboom, H., Carlson, K.H., 2006. Antibiotic resistance genes as emerging contaminants: studies in northern Colorado. Environ. Sci. Technol. 40 7445-50.
10. von Wintersdorff, C.J., Penders, J., van Niekerk, J.M., Mills, N.D., Majumder, S., van Alphen, L.B., Savelkoul, P.H., Wolffs, P.F., 2016. Dissemination of Antimicrobial Resistance in Microbial Ecosystems through Horizontal Gene Transfer. Front. Microbiol. 7 173.
11. Jarlier, V., Nikaido, H., 1994. Mycobacterial cell wall: structure and role in natural resistance to antibiotics. FEMS. Microbiol. Lett. 123 11-8.
12. Meylan, S., Porter, C.B.M., Yang, J.H., Belenky, P., Gutierrez, A., Lobritz, M.A., Park, J., Kim, S.H., Moskowitz, S.M., Collins, J.J., 2017. Carbon Sources Tune Antibiotic Susceptibility in Pseudomonas aeruginosa via Tricarboxylic Acid Cycle Control. Cell. Chem. Biol. 24 195-206.
13. Campos, M.A., Vargas, M.A., Regueiro, V., Llompart, C.M., Albertí, S., Bengoechea, J.A., 2004. Capsule polysaccharide mediates bacterial resistance to antimicrobial peptides. Infect. Immun. 72 7107-14.
14. Frost, L.S., Leplae, R., Summers, A.O., Toussaint, A., 2005. Mobile genetic elements: the agents of open source evolution. Nat. Rev. Microbiol. 3 722-32.
15. Hegstad, K., Mikalsen, T., Coque, T.M., Werner, G., Sundsfjord, A., 2010. Mobile genetic elements and their contribution to the emergence of antimicrobial resistant Enterococcus faecalis and Enterococcus faecium. Clin. Microbiol. Infect. 16 541-54.
16. Pal, C., Bengtsson-Palme, J., Kristiansson, E., Larsson, D.G., 2015. Co-occurrence of resistance genes to antibiotics, biocides and metals reveals novel insights into their co-selection potential. BMC. Genomics. 16 964.
17. Tello, A., Austin, B., Telfer, T.C., 2012. Selective pressure of antibiotic pollution on bacteria of importance to public health. Environ. Health. Perspect. 120 1100-6.
18. Guo, J., Li, J., Chen, H., Bond, P.L., Yuan, Z., 2017. Metagenomic analysis reveals wastewater treatment plants as hotspots of antibiotic resistance genes and mobile genetic elements. Water. Res. 123 468-478.
19. Akiba, M., Senba, H., Otagiri, H., Prabhasankar, V.P., Taniyasu, S., Yamashita, N., Lee, K., Yamamoto, T., Tsutsui, T., Ian Joshua, D., Balakrishna, K., Bairy, I., Iwata, T., Kusumoto, M., Kannan, K., Guruge, K.S., 2015. Impact of wastewater from different sources on the prevalence of antimicrobial-resistant Escherichia coli in sewage treatment plants in South India. Ecotoxicol. Environ. Saf. 115 203-8.
20. Zhang, S., Yang, G., Hou, S., Zhang, T., Li, Z., Liang, F., 2018. Distribution of ARGs and MGEs among glacial soil, permafrost, and sediment using metagenomic analysis. Environ. Pollut. 234 339-346.
21. D'Costa, V.M., King, C.E., Kalan, L., Morar, M., Sung, W.W., Schwarz, C., Froese, D., Zazula, G., Calmels, F., Debruyne, R., Golding, G.B., Poinar, H.N., Wright, G.D., 2011. Antibiotic resistance is ancient. Nature. 477 457-61.
22. Martinez, J.L., 2008. Antibiotics and antibiotic resistance genes in natural environments. Science. 321 365-7.
23. Amann, R.I., Ludwig, W., Schleifer, K.H., 1995. Phylogenetic identification and in situ detection of individual microbial cells without cultivation. Microbiol. Rev. 59 143-69.
24. Xu, Y., Brorson, K., 2003. An overview of quantitative PCR assays for biologicals: quality and safety evaluation. Dev. Biol. (Basel). 113 89-98.
25. Houghton, S.G., Cockerill FR, 3.r.d., 2006. Real-time PCR: overview and applications. Surgery. 139 1-5.
26. Dieffenbach, C.W., Lowe, T.M., Dveksler, G.S., 1993. General concepts for PCR primer design. PCR. Methods. Appl. 3 S30-7.
27. Alberts B, Johnson A, Lewis J, et al. Molecular Biology of the Cell. 4th edition. New York: Garland Science; 2002. References. Available from: https://www.ncbi.nlm.nih.gov/books/NBK26835/
28. Altschul, S.F., Gish, W., Miller, W., Myers, E.W., Lipman, D.J., 1990. Basic local alignment search tool. J. Mol. Biol. 215 403-10.
29. Emmert, D.B., Stoehr, P.J., Stoesser, G., Cameron, G.N., 1994. The European Bioinformatics Institute (EBI) databases. Nucleic. Acids. Res. 22 3445-9.
30. Nurk, S., Meleshko, D., Korobeynikov, A., Pevzner, P.A., 2017. metaSPAdes: a new versatile metagenomic assembler. Genome. Res. 27 824-834.
31. NCBI. Resources. Coordinators., 2017. Database Resources of the National Center for Biotechnology Information. Nucleic. Acids. Res. 45 D12-D17.
32. 2016., Bacterial Antimicrobial Resistance Reference Gene Database
33. O'Niel J., 2016. Tackling drug-resistant infections globally: Final report and recommendations
34. Altschul, S.F., Gish, W., Miller, W., Myers, E.W., Lipman, D.J., 1990. Basic local alignment search tool. J. Mol. Biol. 215 403-10.
35. Rognes, T., Flouri, T., Nichols, B., Quince, C., Mahé, F., 2016. VSEARCH: a versatile open source tool for metagenomics. PeerJ. 4 e2584.
36. Hegde, S.S., Vetting, M.W., Roderick, S.L., Mitchenall, L.A., Maxwell, A., Takiff, H.E., Blanchard, J.S., 2005. A fluoroquinolone resistance protein from Mycobacterium tuberculosis that mimics DNA. Science. 308 1480-3.
37. Kanehisa, M., Goto, S., 2000. KEGG: kyoto encyclopedia of genes and genomes. Nucleic. Acids. Res. 28 27-30.
38. Templeton, N.S., 1992. The polymerase chain reaction. History, methods, and applications. Diagn. Mol. Pathol. 1 58-72.
39. Hugenholtz, P., Tyson, G.W., 2008. Microbiology: metagenomics. Nature. 455 481-3.
40. Schmieder, R., Edwards, R., 2012. Insights into antibiotic resistance through metagenomic approaches. Future. Microbiol. 7 73-89.
41. Hussein, A.I., Ahmed, A.M., Sato, M., Shimamoto, T., 2009. Characterization of integrons and antimicrobial resistance genes in clinical isolates of Gram-negative bacteria from Palestinian hospitals. Microbiol. Immunol. 53 595-602.
42. Chatterjee, S., Datta, S., Roy, S., Ramanan, L., Saha, A., Viswanathan, R., Som, T., Basu, S., 2016. Carbapenem Resistance in Acinetobacter baumannii and Other Acinetobacter spp. Causing Neonatal Sepsis: Focus on NDM-1 and Its Linkage to ISAba125. Front. Microbiol. 7 1126.
43. Fierer, N., Jackson, R.B., 2006. The diversity and biogeography of soil bacterial communities. Proc. Natl. Acad. Sci. U. S. A. 103 626-31.
44. Langelaan, D.G., 1974. Neurological assessment. Nurs. Times. 70 70-2.
45. Hildebrand, F., Meyer, A., Eyre-Walker, A., 2010. Evidence of selection upon genomic GC-content in bacteria. PLoS. Genet. 6 e1001107.
46. Jaurin, B., Grundström, T., 1981. ampC cephalosporinase of Escherichia coli K-12 has a different evolutionary origin from that of beta-lactamases of the penicillinase type. Proc. Natl. Acad. Sci. U. S. A. 78 4897-901.
47. Rush BF, J.r., Humphrey, L., 1967. Primary repair of full thickness excision of the cheek. Am. J. Surg. 114 592-5.
48. Rizzo, L., Manaia, C., Merlin, C., Schwartz, T., Dagot, C., Ploy, M.C., Michael, I., Fatta-Kassinos, D., 2013. Urban wastewater treatment plants as hotspots for antibiotic resistant bacteria and genes spread into the environment: a review. Sci. Total. Environ. 447 345-60.
49. Guo, J., Li, J., Chen, H., Bond, P.L., Yuan, Z., 2017. Metagenomic analysis reveals wastewater treatment plants as hotspots of antibiotic resistance genes and mobile genetic elements. Water. Res. 123 468-478.
50. Wen, Q., Yang, L., Duan, R., Chen, Z., 2016. Monitoring and evaluation of antibiotic resistance genes in four municipal wastewater treatment plants in Harbin, Northeast China. Environ. Pollut. 212 34-40.
51. Chen, J., Michel FC, J.r., Sreevatsan, S., Morrison, M., Yu, Z., 2010. Occurrence and persistence of erythromycin resistance genes (erm) and tetracycline resistance genes (tet) in waste treatment systems on swine farms. Microb. Ecol. 60 479-86.
52. Aminov, R.I., Mackie, R.I., 2007. Evolution and ecology of antibiotic resistance genes. FEMS. Microbiol. Lett. 271 147-61.
53. Lupo, A., Coyne, S., Berendonk, T.U., 2012. Origin and evolution of antibiotic resistance: the common mechanisms of emergence and spread in water bodies. Front. Microbiol. 3 18.
54. Ma, Y., Li, M., Wu, M., Li, Z., Liu, X., 2015. Occurrences and regional distributions of 20 antibiotics in water bodies during groundwater recharge. Sci. Total. Environ. 518-519 498-506.
55. Allen, H.K., Donato, J., Wang, H.H., Cloud-Hansen, K.A., Davies, J., Handelsman, J., 2010. Call of the wild: antibiotic resistance genes in natural environments. Nat. Rev. Microbiol. 8 251-9.
56. Xu, J., Xu, Y., Wang, H., Guo, C., Qiu, H., He, Y., Zhang, Y., Li, X., Meng, W., 2015. Occurrence of antibiotics and antibiotic resistance genes in a sewage treatment plant and its effluent-receiving river. Chemosphere. 119 1379-1385.
57. Munir, M., Wong, K., Xagoraraki, I., 2011. Release of antibiotic resistant bacteria and genes in the effluent and biosolids of five wastewater utilities in Michigan. Water. Res. 45 681-93.
58. Rodriguez-R, L.M., Gunturu, S., Tiedje, J.M., Cole, J.R., Konstantinidis, K.T., 2018. Nonpareil 3: Fast Estimation of Metagenomic Coverage and Sequence Diversity. mSystems. 3 .
59. Cytyrn E. 2013. The soil resistome: The anthropogenic, the native, and the unknown. Soil Biol Biochem. 63 18-23.
60. Rodriguez-Mozaz, S., Chamorro, S., Marti, E., Huerta, B., Gros, M., SA nchez-Melsió, A., Borrego, C.M., Barceló, D., Balcázar, J.L., 2015. Occurrence of antibiotics and antibiotic resistance genes in hospital and urban wastewaters and their impact on the receiving river. Water. Res. 69 234-242.
61. Zhu, Y.G., Johnson, T.A., Su, J.Q., Qiao, M., Guo, G.X., Stedtfeld, R.D., Hashsham, S.A., Tiedje, J.M., 2013. Diverse and abundant antibiotic resistance genes in Chinese swine farms. Proc. Natl. Acad. Sci. U. S. A. 110 3435-40.
62. Threlfall, E.J., Ward, L.R., Frost, J.A., Willshaw, G.A., 2000. The emergence and spread of antibiotic resistance in food-borne bacteria. Int. J. Food. Microbiol. 62 1-5.
63. Szczepanowski, R., Linke, B., Krahn, I., Gartemann, K.H., Gützkow, T., Eichler, W., Pühler, A., Schluter, A., 2009. Detection of 140 clinically relevant antibiotic-resistance genes in the plasmid metagenome of wastewater treatment plant bacteria showing reduced susceptibility to selected antibiotics. Microbiology. 155 2306-19.
64. Tian, B., Fadhil, N.H., Powell, J.E., Kwong, W.K., Moran, N.A., 2012. Long-term exposure to antibiotics has caused accumulation of resistance determinants in the gut microbiota of honeybees. MBio. 3 .
65. Wanninger, S., Donati, M., Di Francesco, A., Hässig, M., Hoffmann, K., Seth-Smith, H.M., Marti, H., Borel, N., 2016. Selective Pressure Promotes Tetracycline Resistance of Chlamydia Suis in Fattening Pigs. PLoS. One. 11 e0166917.

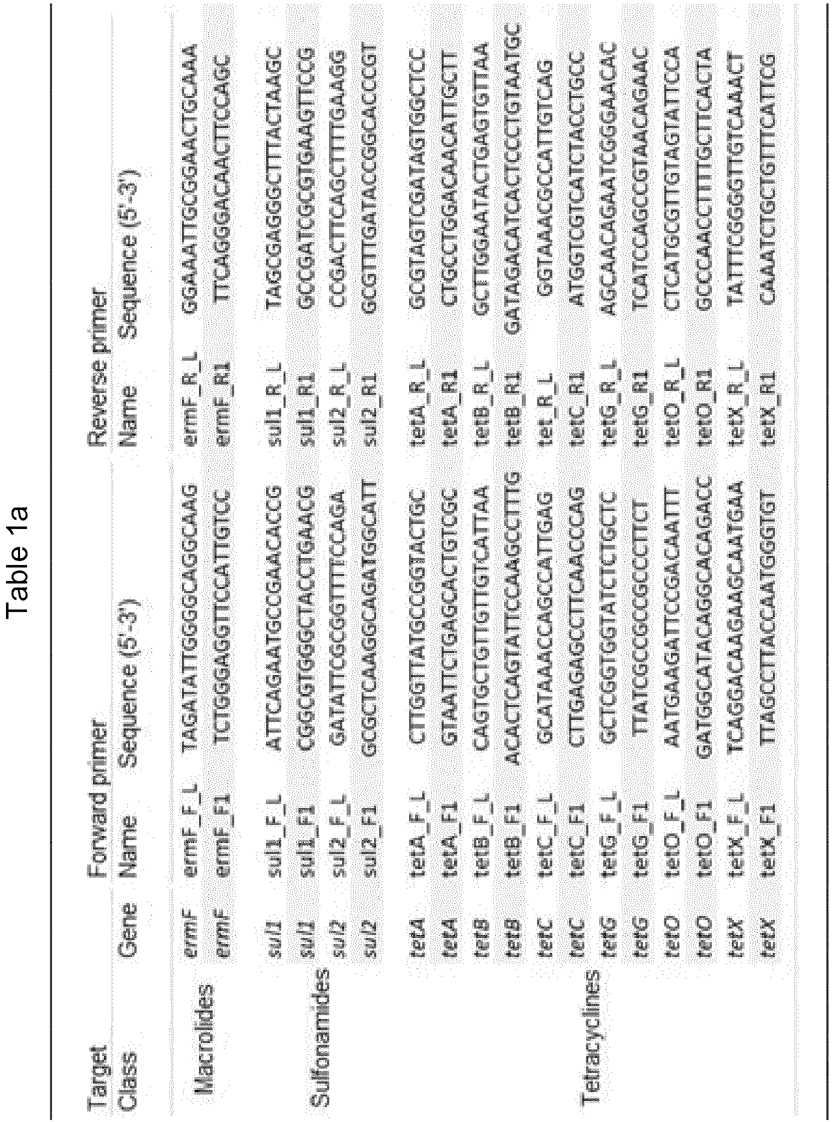

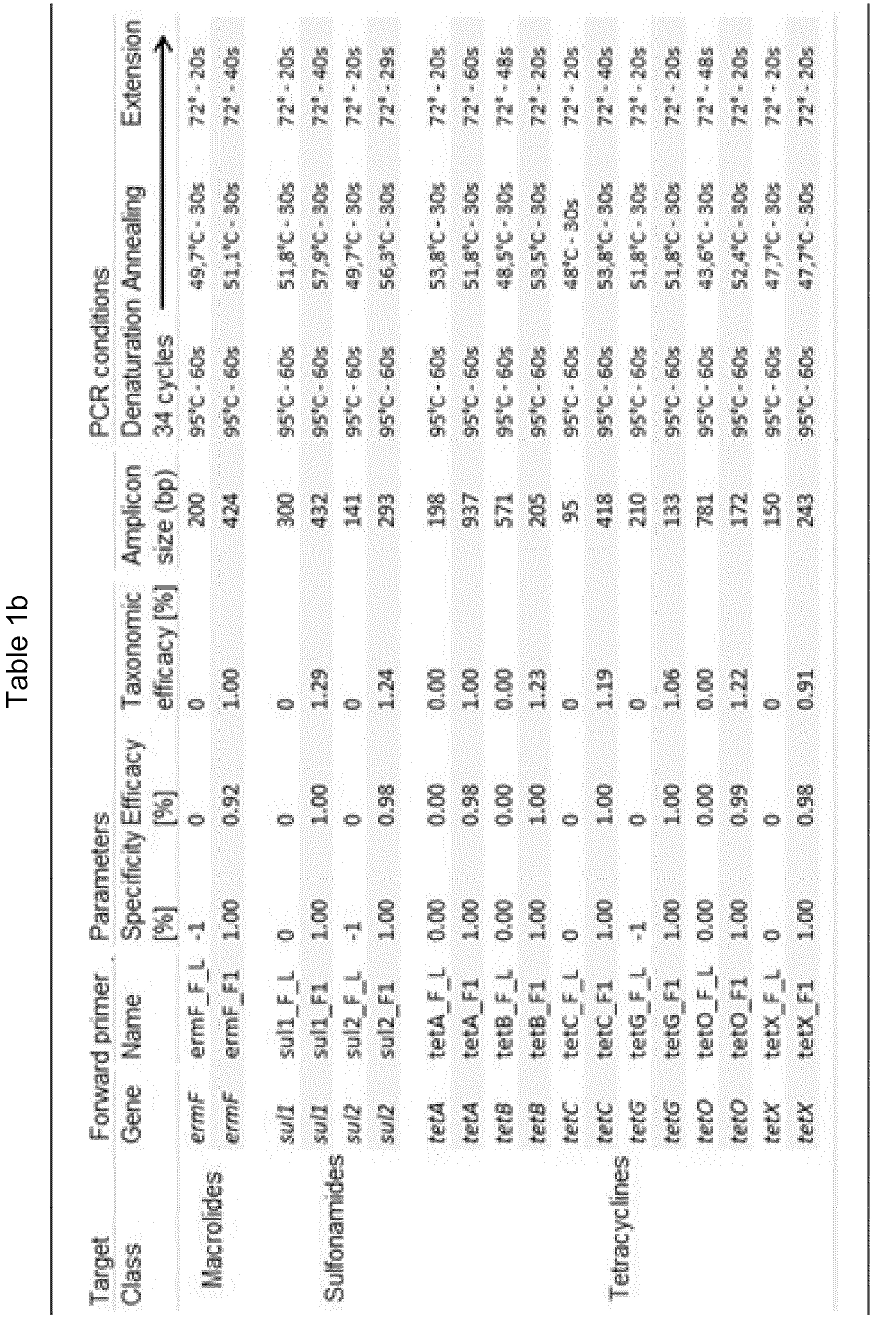

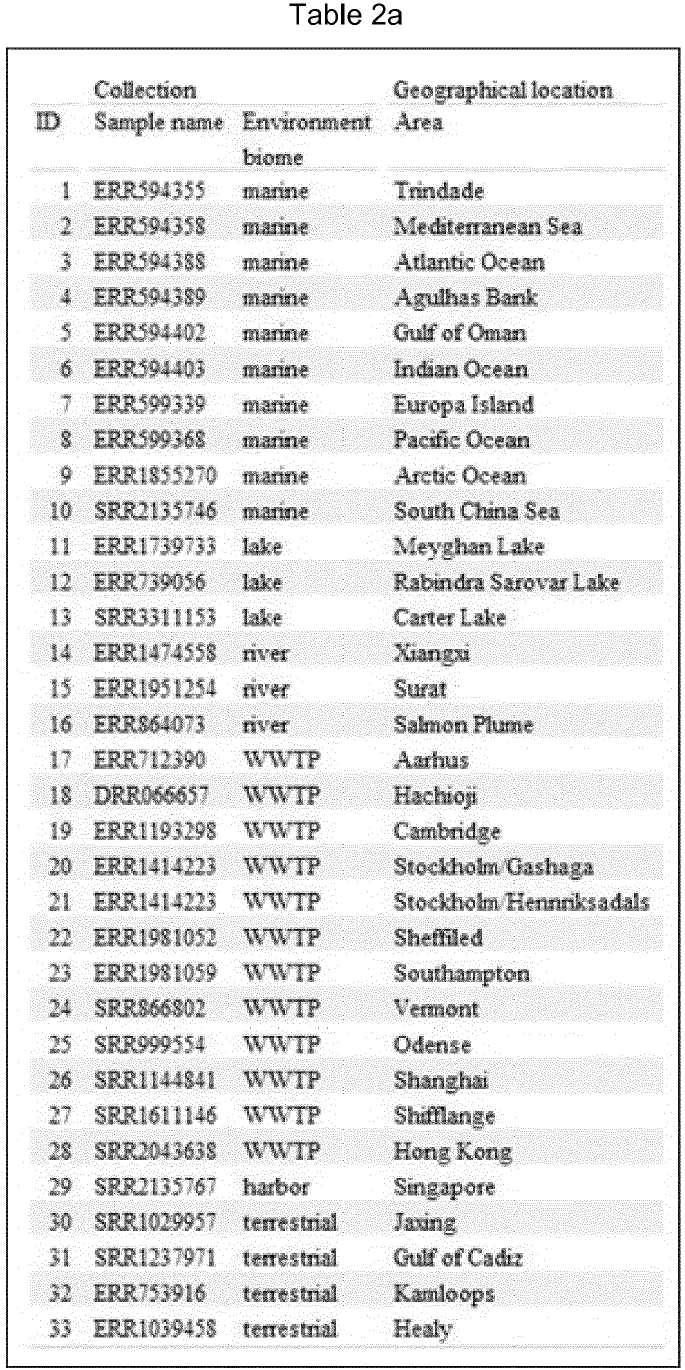

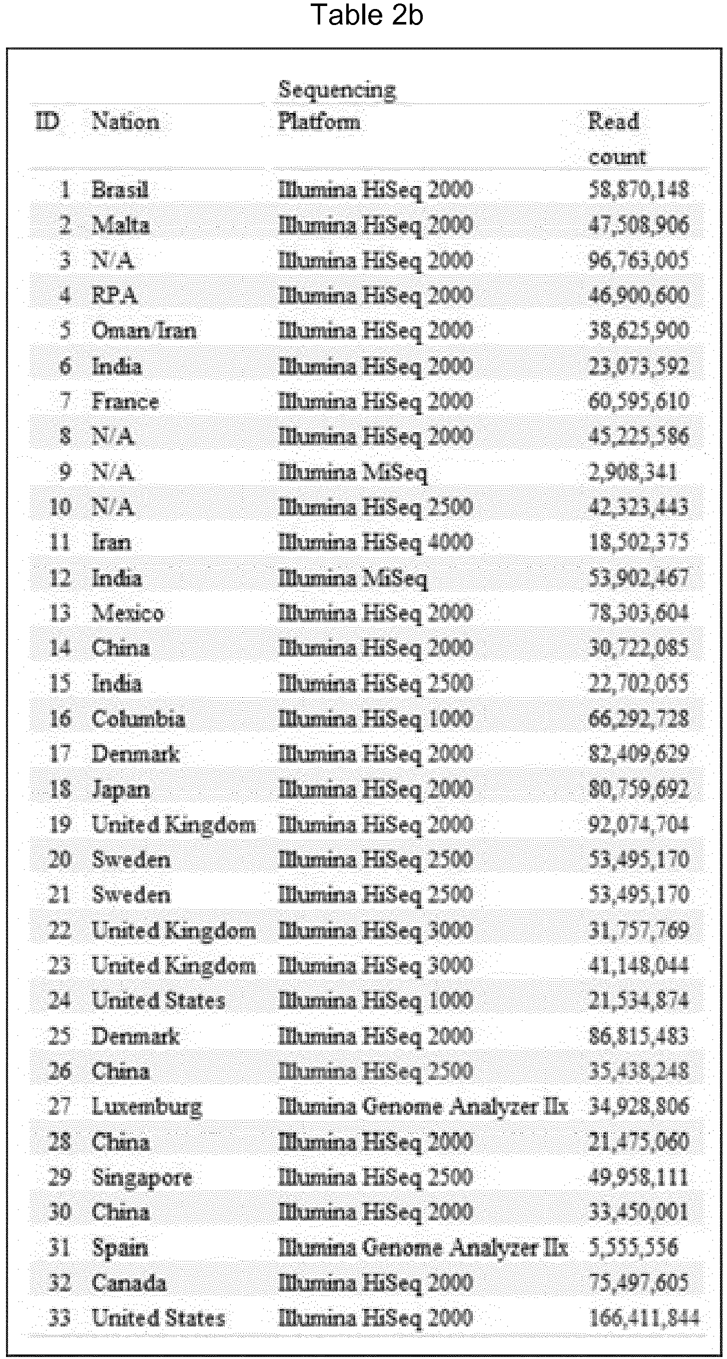

## Claims

1. A method of selecting PCR primers, preferably for detection of antibiotic resistance genes, comprising the following computer-implemented steps:
a) providing a first reference gene sequence database comprising first gene sequences;
b) providing a second reference gene sequence database comprising second gene sequences;
c) searching said first gene sequences in said second reference gene sequence database;
d) creating a third redundant gene sequence database comprising the gene sequences found as the result of step c);
e) creating a third non-redundant gene sequence database by selecting all and only unique gene sequences from said third redundant gene sequence database;
f) providing a test primer database, comprising primer pairs for amplifying gene sequences;
g) performing *in silico* PCR using as a matrix said second reference gene sequence database and said test primer database;
h) counting the number P of products obtained in the step g) for each primer pair from said test primer database;
i) for each of the P products, searching for it in said third redundant gene sequence database and counting the number of products found therein and correct products CP;
j) performing *in silico* PCR using as a matrix said third non-redundant gene sequence database and said test primer database;
k) counting the number RP of reference products obtained in the step j) for each primer pair from said test primer database;
l) for each primer pair from said test primer database: calculating the taxonomic diversity of products as the number PT of taxons from which the products of step g) originated and the reference taxonomic diversity as the number RT of taxons corresponding to all variants of a particular gene sequence from said first reference gene sequence database found in said third redundant gene sequence database;
m) for each primer pair from said test primer database:
calculating the primers specificity parameter S as the ratio of correct products CP to the number P of products obtained in the step g); S=CP/P;
calculating the primers efficacy parameter E as the ratio of reference products RP to the number NRER of the non-redundant variants of a particular gene in said third non-redundant gene sequence database; E=RP/NRER;
calculating the primers taxonomic efficacy parameter TE as the ratio of the taxonomic diversity of products PT to the reference taxonomic diversity RT; TE=PT/RT;
n) for each primer pair from said test primer database: assigning parameters of the primers specificity parameter S, the primers efficacy parameter E and the primers taxonomic efficacy parameter TE to said primer pair;
o) selecting primer pairs according to their values of the primers specificity parameter S and/or the primers efficacy parameter E and/or the primers taxonomic efficacy parameter TE.

2. The method according to claim 1, wherein said first reference gene sequence database is the Bacterial Antimicrobial Resistance Reference Gene Database (BARRGDB).

3. The method according to claim 1 or 2, wherein said second reference gene sequence database is the National Center for Biotechnology Information Nucleotide Database (NCBI NT).

4. The method according to claim 1, 2 or 3, wherein step c) is performed using the BLASTn algorithm, preferably with the threshold of 90% sequence coverage and 90% sequence identity.

5. The method according to any one of the claims 1 to 4, wherein step e) consists in dereplicating said third redundant gene sequence database, preferably using the VSEARCH tool.

6. The method according to any one of the claims 1 to 5, wherein step g) is performed using ePCT tool, preferably with the following parameters: . word size: 6, e-value: 10000, reward for positive match: 1, penalty for mismatch: -2, penalty for gap opening: 5, penalty for gap extension: 3, maximum number of aligned sequences to keep: 10000, minimum query coverage per subject: 75.

7. The method according to any one of the claims 1 to 6, wherein said taxons correspond to bacterial families.

8. The method according to any one of the claims 1 to 8, wherein in step o) primer pairs having higher values of the primers specificity parameter S and/or higher values of the primers efficacy parameter E and/or higher values of the primers taxonomic efficacy parameter TE are selected.

9. The method according to any one of the claims 1 to 9, wherein said first reference gene sequence database and/or said second reference gene sequence database and/or said test primer database is created based on a biological sample.

10. The method according to any one of the claims 1 to 10, wherein the primer pair or primer pairs selected is step o) are applied in a laboratory environment, preferably to amplify target gene sequence(s).
